(19) Europäisches Patentamt
European Patent Office
Office européen des brevets

(11) Publication number: **0 151 760**
**A1**

(12) # EUROPEAN PATENT APPLICATION

(21) Application number: 84115551.8

(22) Date of filing: 15.12.84

(51) Int. Cl.[4]: C 12 N 15/00
C 12 P 21/00, C 07 H 21/04
C 12 P 19/34, C 07 K 15/26
//C12R1/07, C12R1/125

(30) Priority: 06.01.84 JP 1204/84
27.03.84 JP 60375/84
27.09.84 JP 203772/84

(43) Date of publication of application: 21.08.85 Bulletin 85/34

(84) Designated Contracting States: AT BE CH DE FR GB IT LI LU NL SE

(71) Applicant: Takeda Chemical Industries, Ltd.
27, Doshomachi 2-chome Higashi-ku
Osaka-shi Osaka, 541(JP)

(72) Inventor: Kikuchi, Masakazu
4-16 Higashitokiwadai 7-chome Toyono-cho
Toyono-gun Osaka 563-01(JP)

(72) Inventor: Nakahama, Kazuo
15-59, Nishinokyo
Nagaokakyo Kyoto 617(JP)

(72) Inventor: Yoshimura, Koji
6-31, Zuiko 1-chome Higashiyodogawa-ku
Osaka 533(JP)

(74) Representative: von Kreisler, Alek, Dipl.-Chem. et al,
Deichmannhaus am Hauptbahnhof
D-5000 Köln 1(DE)

(54) **Novel DNA and use thereof.**

(57) A novel DNA containing a promoter which is preferably a neutral protease promoter, and a signal peptide-encoding region of the neutral protease gene. The DNA may further contain a neutral protease-encoding region of the neutral protease gene so far as a portion of its nucleotides containing the 3' end nucleltide is missing. A transformant of a strain of the genus *Bacillus* transformed with the DNA which further contains a desired peptide-encoding gene other than that of a neutral protease, can produce the peptide extracellularly.

EP 0 151 760 A1

0151760

# NOVEL DNA AND USE THEREOF

This invention relates to a novel DNA and its use.

Many studies on a recombinant DNAs have been so far made with *Escherichia coli* and a number of foreign genes have already been expressed in *Escherichia coli*. However, since the product expressed by this method is accumulated within the cells of *Escherichia coli*, much time and labor are required to recover the desired product from the cells. Especially, the extraction of the product from the cells and the subsquent purification encounter with a number of difficulties. It is almost impossible to obtain the product in the pure and complete form.

From the past, a microorganism belonging to the genus *Bacillus* has been utilized for industrial use as a microorganism for producing various exo-enzymes. It is considered to be possible to secrete the desired protein as the gene product by cloning a promoter and a DNA region coding for the signal peptide of the exo-enzyme gene, ligating the structural gene of the desired protein downstream from the above-mentioned DNA region, and incorporating the resultant DNA into a strain of *Bacillus subtilis*. In the microorganism belonging to the genus *Bacillus*, the protein secreted is considered to be synthesized first as a precursor having a signal peptide consisting of 20-30 amino acids at the amino terminal, the signal peptide being subsequently cleaved by a signal peptidase to give the protein. It has been reported that the α-amylase gene of Bacillus amyloliquefaciens [I. Palva et al ; Gene, 22, 229 (1983)] , the penicillinase gene of Bacillus licheniformis [S. Chang, et al: Molecular Cloning and Gene Regulation in Bacilli, Academic Press, pp659, (1982)] and the α-amylase gene of Bacillus subtilis [H. Yamazaki et al: J. Bacteriol, 156, 327, (1983)] have been already cloned and a number of foreign proteins have been reported to be secreted by utilizing these signal peptides. It has been also reported that the alkaline protease gene of B. amyloliquefaciens has been cloned and the base sequence has been determined [J.A. Wells, et al, Nucleic Acids Res. 11, 7911, (1983)]. Recently, it has been further reported that the neutral protease gene of B. amyloliquefaciens has been cloned [N. Tomioka, et al. Abstracts of Papers, Annual Meeting of the Agricultural Chemical Society of Japan, 1983, pp33]. But the gene has been found not to encode for a neutral protease by determining its base sequence [Y. Furutani: Text for 1st Symposium on Research and Development project of Basic Technology for Future Industries, p223, 1983]

In the conventional method of expressing a desired protein with the use of the signal peptide encoding region of an exo-enzyme gene of a strain of the genus Bacillus , much time and labor are required to ligate a DNA encoding for the desired protein and, moreover, it is very difficult to express the desired protein directly, because there is no suitable recognition site near the 3' end of the region coding for the signal peptide. Further, much time is required to express the desired protein by culturing a microorganism carrying the plasmid having incorporated therein a DNA coding for the desired protein and, hence, there is a great demand for an improved expression system capable of producing the desired protein product within a shortened period of time. The present inventors have succeeded in cloning a novel, neutral protease gene-carrying DNA whose restriction enzyme map differs from that of known DNAs. By ligating the novel DNA with a vector and incorporating the ligated product into a microorganism belonging to the genus Bacillus, an excellent transformant capable of producing a neutral protease at a high rate has been found to be obtained. It has now been revealed that a useful secretion vector can be constructed by utilizing the signal peptide-encoding region of the DNA.

In accordance with one aspect of the present invention, there is provided a DNA comprising a promoter, and the neutral protease gene containing a signal peptide encoding region and having at least a portion of its neutral protease encoding region cut away, said cut away portion being the 3' end nucleotide, or the 3' end nucleotide and one or more contignuous linked thereto.

In another aspect, the present invention provides a method of producing a DNA, comprising (a) digesting a first DNA fragment containing the neutral protease gene having a promoter, a signal peptide-encoding region and a neutral protease-encoding region with a restriction enzyme to cut away a portion of the neutral protease-encoding region, said cut away portion being the 3' end nucleotide, or the 3' end nucleotide and one or more contiguous necleotides linked thereto; or (b) joining a second DNA fragment having promoter activity to a third DNA fragment containing a signal peptide-encoding region of the neutral protease gene and a neutral protease-encoding region, at least a portion of said neutral protease-encoding region being missing, said missing portion being the 3' end nucleotide, or the 3' end nucleotide and one or more contiguous nucleotides linked thereto; and, if necessary, (c) joining a desired protein-encoding gene at a position downstream from said signal peptide-encoding region, whereby obtaining a DNA comprising a promoter, and the neutral protease gene containing a signal peptide-encoding region and having at least a portion of its neutral protease-encoding region cut away, said cut away portion being the 3' end nucleotide, or the 3' end nucleotide and one or more contiguous necleotides linked thereto, optionally having the desired protein-encoding gene at a position downstream from said signal peptide-encoding region.

In a further aspect, the present invention provides a transformant of a strain of microorganism belonging to the genus Bacillus and transformed with a DNA comprising a promoter, and the neutral protease gene containing a signal peptide encoding region and having at least a portion of its neutral protease encoding region cut away, said cut away portion being the 3' end nucleotide, or the 3' end nucleotide and one or more contiguous linked thereto.

The present invention also provides a process for the production of a peptide, comprising cultivating a transformant obtained by transforming a strain of microorganism belonging to the genus Bacillus with a DNA including a promoter, the neutral protease gene containing a signal peptide encoding region, and a gene coding for a peptide other than the neutral protease, said neutral protease gene having at least a portion of its neutral protease encoding region cut away, said cut away portion being the 3' end nucleotide, or the 3' end nucleotide and one or more contiguous nucleotides linked thereto, whereby said peptide is secreted outside the microorganism.

The DNA containing a signal peptide encoding region of the neutral protease may be any of those derived from microorganisms capable of producing a neutral protease, for instance, microorganisms belonging to the genus Bacillus, such as B. amyloliquefaciens.

The promoter used in the present invention is of a type which is capable of functioning as a promoter in a microorganism belonging to the genus Bacillus. Examples of suitable promoters include those derived from gram-positive microorganisms such as those belonging to the genus Staphylococcus and the genus Bacillus. Illustrative of the microorganisms belonging to the genus Bacillus are B.subtilis, B. pumilus and B. amyloliquefaciens. Promoter which are known per se (for instance veg promoter, SPO1 promoter and SPO2 promoter) can be also used.

The number of the promoter used may be one or more, which are connected in directly repeated form.

The promoter and the DNA coding for the signal peptide of the neutral protease gene can be obtained from the chromosomal DNA of the above-mentioned microorganisms. The chromosomal DNA can be prepared from the microorganism by a per se known method, for instance the method of Lovett et al [Methods in Enzyomology, 68, 342 (1979)].

As a host-vector system in which the DNA fragment containing the neutral protease gene is cloned from a chromosomal DNA, any of those capable of confirming the presence of the neutral protease gene on the chromosomal DNA fragment inserted into the vector can be used and, preferably, there may be mentioned a Bacillus subtilis as a host-vector system. The cloning of a DNA fragment containing the neutral protease gene with the use of a vector may be conducted by cleaving the vector with, for

example, a restriction enzyme, joining the DNA fragment to the cleaved vector with a DNA ligase, and transforming a microorganism having no or poor ability to produce a neutral protease with the resultant DNA. The desired cloned product may be obtained from the strains selected from the resulting transformants and capable of producing the neutral protease. When the vector has a drug resistant gene and an insertion inactivation site located on the gene, it is possible to utilize the sensitivity to the drug as an indicator for the selection of the said strains by inserting the DNA fragment at the said inactivation site. Plasmids pBTM119 and pUB110 are specific examples of such vectors.

A strain having the ability to produce a neutral protease as a exo-enzyme can form a halo around a colony when cultivated, for example, on the agar plate containing 1% casein. This property may be utilized for the selection of the desired strain from the trans- formants. An example of a strain having a low ability to produce a neutral protease is B. substilis 1A274 [The Bacillus Genetic Stock Center: Catalog of Strains, 2nd edition (1982)]. Any conventional method may be used for the transformation of a strain of the genus Bacillus. Illustrative of suitable transformation methods are the protplast method [S. chang & S. N. Cohen : Mol. Gen. Genet., 168, 111 (1979)] and the competent method [D. Dubnau & R. D. Abelson, J. Mol. Biol., 56, 209(1971)].

A DNA containing the inserted neutral protease gene may be isolated by extracting the plasmid with the transformant cells, digesting the plasmid by, for example, a restriction enzyme, followed by separation by, for example, agarose gel electrophoresis or polyacrylamide gel electrophresis. Such an isolation technique is well known in the art and described in detail in articles [Methods in Enzymology, 68, (1979); and Molecular Cloning (1982), Cold Spring Harbor Lab.].

The base sequence of a DNA containing the neutral protease gene may be determined by, for instance, the dideoxynucleotide synthetic chain termination method [Proc. Natl. Acad. Sci., USA, 74, 5463 (1977)] or the method of Maxam-Gilbert [A. M. Maxam & W. Gilbert; Proc. Natl. Acad. Sci., USA, 74, 560 (1977)] or the like. The position of the neutral protease gene in the DNA may be determined by comparing the amino acid sequence translated from the determined base sequence of the DNA with the known amino acid sequence of a neutral protease [P. L. Levy et al: Proc. Natl. Acad. Sci.,USA, 72, 4341 (1975)] . Whether or not the neutral protease gene is contained in the DNA may be determined from the amino acid sequence of the matured neutral protease produced by the host transformed with a plasmid containing the DNA.

The signal peptide encoding region of the neutral protease gene includes a polynucleotide coding for the peptide represented by the following amino acid sequence:

Met - Gly - Leu - Gly - Lys - Lys - Leu - Ser - Val - Ala - Val - Ala - Ala - Ser - Phe - Met - Ser - Leu - Thr - Ile - Ser - Leu - Pro - Gly - Val - Gln - X (wherein X stands for Ala or Ala-Ala). The polynucleotide may have any combination of codons as long as it can code for the peptide having the above amino acid sequence. A polynucleotide coding for a peptide in which an amino acid or acids are inserted into, deleted from or substituted for an amino acid or acids of the peptide of the above amino acid sequence, may be used as a region coding for the signal peptide, as long as the peptide serves to function as the signal peptide.

The DNA containing the neutral protease gene which has a region coding for the signal peptide and in which a part or whole of the region coding for a neutral protease is missing (the missing portion includes one or more contiguously arranged nucleotides containing the 3' end nucleotide) may be obtained by completely or partially digesting a DNA containing the neutral protease gene with, for example, a restriction enzyme. Such a DNA may be also obtained by completely or partially digesting a DNA containing the neutral protease gene with a restriction enzyme to form a smaller sized DNA fragment containing the neutral protease gene, subcloning the DNA fragment and digesting completely or partially the cloned product with a restriction enzyme.

Sub-cloning may be carried out by cloning the smaller sized DNA fragment on a vector in the same manner as that in cloning of the neutral protease gene. Whether or not the neutral protease gene is cloned on the vector may be determined from the presence or absence of the halo formed around the colony as a result of the decomposition of casein by the transformant. Examples of such vectors include plasmids from Bacillus subtilis, such as plasmids pBTB119, pUB110 and pPL603.

The DNA containing the region coding for a signal peptide of the neutral protease gene may be any of those chemically fully or partially synthesized by a per se known method such as the triester method [Proc. Natl. Acad. Sci., USA, 75, 5765 (1978)].

As the vector (promoter cloning vector) for use in cloning a DNA fragment having a promoter activity with the use of a chromosomal DNA, there may be used any of those plasmids into which a chromosomal DNA fragment derived from a strain of the genus Bacillus can be inserted at, for example, the restriction enzyme cleavage site and in which the presence of the promoter in said fragment can be confirmed. For instance, there may be mentioned a plasmid containing a promoter portion deficient gene. Specifically, the plasmid pPL603 (pBTM126) is a typical example [J. Bacteriol., 146, 1162 (1981)].

Using the vector into which a chromosomal DNA fragment has been inserted, a strain of the genus Bacillus is transformed. A plasmid is obtained from the transformant by any conventional method, and the thus obtained plasmid is digested with a restriction enzyme, followed by separation by polyacrylamide gel electrophoresis or agarose gel electrophoresis, whereby a DNA fragment having a promoter activity. The base sequence of the DNA fragment thus isolated may be determined by a per se known method such as the dideoxynucleotide synthetic chain termination method (vide supra) or the method of Maxam - Gilbert (vide supra).

The DNA fragment having a promoter activity may also be chemically synthesized by a per se known method, such as the phospho-triester method. Such a synthetic DNA fragment may be used for the purpose of the present invention.

The DNA containing a signal peptide-encoding region of the neutral protease gene and the DNA fragment having a promoter activity are joined with each other by a per se known method and the resulting DNA is inserted into a plasmid, thereby to obtain a vector having both a promoter and a signal peptide-encoding region of the neutral protease gene. A vector containing both a promoter and a signal peptide-encoding region of the neutral protease gene may be also constructed by inserting one of the DNA fragment containing a signal peptide-encoding region of the neutral protease gene and the DNA fragment having a promoter activity, into the vector containing the other one of the DNA fragments. When a neutral protease promoter is used as the promoter for the construction of the vector, it is not necessary to cleave the site between the promoter region and the signal peptide-encoding region of the neutral protease gene.

The desired protein or peptide may be obtained by transforming a strain of the genus Bacillus with a DNA having the promoter required for the initiation of transcription, a ribosome binding site (SD sequence) located downstream of the promoter, signal peptide encoding region of the neutral protease gene located downstream of the SD sequence and a gene coding for the desired protein or peptide located downstream of the signal peptide encoding region, and cultivating the transformant thus obtained.

The SD sequence may be any of those capable of functioning in a microorganism belonging to the genus Bacillus such as Bacillus subtilis, inclusive of several known ones [J. R. McLaughlin et al: J. Biol. Chem., 256, 11283 (1981), C. P. Moran. Jr. et al: Mol. Gen. Genetics, 186, 339 (1982)]. An oligonucleotide containing an SD sequence can be isolated from a chromosomal DNA or chemically synthesized such an oligonucleotide by a per se known method, for instance the phospho-triester method (vide supra)., By inserting the oligonucleotide into a vector containing a promoter and a signal peptide encoding of the neutral protease gene at a position downstream from the promoter, the desired expression vector may be constructed.

The gene coding for desired protein may be ligated at a position just behind the signal peptide encoding region of the neutral protease gene, just before the region coding for a mature neutral protease or in the neutral protease gene ligated. In order to obtain the desired protein, it is preferred that the DNA coding for the desired protein is joined at a position just behind the signal peptide encoding region of the neutral protease gene or just before the region coding for a mature neutral protease. The base sequence (propeptide-encoding region) starting from just behind of the region coding for a signal peptide of a neutral protease and ending at just before the region coding for a neutral protease (from the 917th base in Fig. 6 to the 63th base in Fig. 7) is not specifically limited as long as the resultant DNA may give the desired protein. Thus, a nucleotide or nucleotides may be inserted within the region. Alternately, a nucleotide or nucleotides in the region can be deleted or substituted with other nucleotide or nucleotides.

It is desirable that the gene used to be expressed does not contain any intervening (intron) sequence and that the base sequence of the said gene has been demonstrated. Such genes may be genes isolated from chromosomes, complementary DNAs obtained from m-RNAs, chemically synthesized genes, and semi-synthetic genes, etc. More specifically, there may be mentioned the hepatitis B virus (HBV) surface antigen gene, HBV core antigen gene, immunoglobulin E gene, human growth hormone gene, the interleukine-2 gene, the immune interferon gene, penicillinase gene and so forth. A part or whole of such genes or genes having a plurality of such genes linked with each other may be used. In constructing expression plasmids by inserting these genes into expression vectors, an appropriate synthetic oligonucleotide may be joined to the genes, if necessary.

When transforming the genus Bacillus with the plasmid thus obtained, the host is not particularly limited to specific strains, but includes, for instance B. subtilis BGSC1A1, BGSC1A274 [The Bacillus Genetic Stock Center : Catalog of Strains, 2nd edition (1982)]. Above all, the strains having a low or no ability to produce a protease may be preferably used.

The medium used for cultivating the transformant may be any of media in which the transformant can grow and produce the desired protein.

As a carbon source for such a medium, glucose, sucrose, glycerol, starch, dextrin, molasses and organic acids, for instance, may be used. And as a nitrogen source, an organic nitrogen source such as casein, pepton, nutrient extract, yeast extract, casamino acids [Difco (USA)], N.Z-amine A [Shefield (USA)], soybean meal or peanut powder and an inorganic nitrogen source such as ammonium sulfate, ammonium chloride and ammonium nitrate may be used. Minerals such as calcium salts, magnesium salts, potassium salts, sodium salts, phosphates, manganese salts, iron salts and cobalt salts may be added to the medium if necessary. When the strain used requires specific nutrient, the nutrients such as amino acids, vitamines and nucleic acid bases, may be added to the medium.

If necessary, an antifoaming agent (for instance, a soy bean oil or a lard oil) may also be added to the medium.

Further, if necessary for effecting cultivation, an antibiotic, for instance chloramphenicol, may be added to the medium.

The cultivation temperature is generally from about 15 °C to 42 °C, preferably from about 24 °C to 37 °C, and the initial pH of the medium is about 5.0 to 9.0, preferably about 6.5 to 7.5 and the cultivation time is generally about 3 to 72 hours, preferably about 5 to 48 hours.

The desired protein may be purified by a per se known protein purifying method, after the removal of the cells by a per se known method such as by centrifugation.

The present invention contemplates the provision of a novel promoter derived from the genus Bacillus.

It is known that, in some strains of the genus Bacillus, different RNA polymerases act in different stages of growth of the cells and recognize different promoter regions [Nature, 302, 800 (1983)]. Namely, it is known that the RNA polymerase (such as $E\sigma^{55}$) in vegetative cells recognizes a promoter region which is different from that recognized by the RNA polymerase (such as $E\sigma^{32}$ or $E\sigma^{37}$) in sporulation cells.

The present inventors have made an intimate study on the expression mechanism of the neutral protease gene and, as a result, they have found that the promoter (the neutral protease promoter) for expressing the neutral protease gene has an excellent property.

Thus, the present invention is to provide a promoter whose -35 region and -10 region are TTGCAG and TATTAT ($E\sigma^{55}$), respectively, whose -35 region and -10 region are AGTTTT and GAGATTGCT ($E\sigma^{37}$), respectively, or whose -35 region and -10 region are AATTC and TAGTTTTATA ($E\sigma^{32}$), respectively.

The promoter may be obtained from the chromosome of the genus Bacillus having an ability to produce a neutral protease, such as B. amyloliquefaciens. The promoter may be of a type which is chemically synthesized by a per se known method.

The desired protein may be obtained by transforming the genus Bacillus with the DNA having a gene coding for the desired protein gene located downstream from the promoter of the present invention and cultivating the transformant thus obtained. A DNA having a signal peptide encoding region of the neutral protease gene inserted between the promoter and the desired protein gene may also be used in order to produce the desired protein.

Though the number of the RNA polymerase recognition site in the promoter is at least one, it is preferred that promoters having two or more RNA polymerase recognition sites are used so that the promoters can function as promoters at various growth stages (for instance, vegetative cell, sporulation cell)

As the desired protein may be produced and secreted extracellularly by using the DNA according to the present invention, the purification of the protein may be effected much easier as compared with the extraction of the protein from the cells. Since the neutral protease gene has restriction enzyme recognition sites near the 3' end of the region coding for a signal peptide and near the 5' end of the region coding for a matured protease, it is relatively easy to insert the DNA coding for the desired gene and to cut out the inserted DNA. By using the DNA according to the present invention, a short cultivation time is sufficient to produce the desired protein.

The following reference examples, working examples and the accompanying drawings will further illustrate the present invention. It is to be noted, however, that such examples are by no means limitative of the present invention.

In the drawings:

Figs. 1 and 2 show the restriction enzyme maps of

the plasmid pBTM119 and the plasmid pBTM127, respectively;

Fig. 3 shows the procedures for constructing the plasmids pBTM508 and pBTM515;

Fig. 4 shows the procedure for constructing the plasmid pBTM 523;

Fig. 5 shows the restricted enzyme map of the DNA fregment (C fregment in Example 3) containing the neutral protease gene obtained from <u>B. amyloliquefaciens</u> (IFO-14141), the symbols Bg, T, Sa, Ha, S, A, Bc and H indicating BglII, TaqI, SacII, HaeIII, Sau3AI, AccI, BclI and HindIII, respectively;

Fig. 6 shows the base sequence around the 5' end of the DNA fregment (C fragment in Example 3) containing the neutral protease gene and the amino acid sequence translated from the base sequence;

Fig. 7 shows the base sequence around the 3' end of the DNA fragment and the amino acid sequence translated from the base sequence, the symbol TAA indicating the stop codon;

Fig. 8 shows the procedure for constructing the plasmid pTEX101, the symbols nprA and nprA' indicating the neutral protease and the neutral protease gene deficient in the mature protein-encoding region, respectively;

Fig. 9 shows the procedure for constructing the plasmid pBTM150, the symbols trp-p and IFN-$\gamma$ indicating the tryptophan promoter and the human immune interferon gene;

Fig. 10 shows the procedure for construction the plasmid pENX374;

Fig. 11 shows the base sequence around the 5' end of the penicillinase gene and the amino acid sequence of an N-end portion;

Fig. 12 shows the base sequence around the 5' end of the penicillinase gene included in the plasmid pENX374 and the amino acid sequence translated from the base sequence;

Fig. 13 shows the base sequence around the 5' end of the penicillinase gene included in the plasmid pENX606 and the amino acid sequence translated from the base sequence;

Fig. 14 shows the procedure for constructing the plasmid pTEX201 and Fig. 15 shows the procedure for constructing the plasmid pTEX202;

Reference Example 1

Construction of Cloning Vector pBTM119:

The Cloning vector pBTM119 was constructed by the method described in Japanese Published Unexamined Patent Application No. 59 - 55879.

i) Preparation of Drug Resistant Plasmid:

Chloramphenicol resistant *Staphylococcus epidermidis* FS 5030 [deposited with The Institute for Fermentation, Osaka, under IFO-14203 and also deposited with Fermentation Research Institute, Agency of Industrial Science and Technology, Ministry of International Trade and Industry (FRI) under FERM BP-670 according to the Budapest Treaty] was cultured at 30 °C for 18 hours with shaking in a test tube with 5 ml of L medium containing 0.5% yeast extract and 0.5% NaCl (pH 7.2). 20 ml (corresponding to the sum of the contents in 4 test tubes) of the resultant culture solution was centrifuged. The resultant cells were washed with 10 mM EDTA at pH 8.0, and were then suspended in 2 ml of TBL-1 lytic enzyme solution (1 mg/ml, 10 mM EDTA (pH8.0)) and kept warm at 37°C for 30 mins. After adding 0.2 N NaOH solution containing 4 ml of 1% sodium lauryl sulfate to the suspension, the mixture was allowed to stand at 0°C for 5 min and then mixed with 6 ml of a 3 M sodium acetate solution (pH 4.8). The resulting mixture, after being allowed to stand at 0°C for 60 min, was centrifuged to obtain a supernatant, to which was added two volumes of cold ethanol. After being allowed to stand at -20°C overnight, the mixture was again centrifuged. The precipitate was dissolved in 0.4 ml of 0.4% Sarkosyl solution, to which were added 2.4 ml of TES buffer solution (30 mM Tris-HCl buffer solution (pH 8.0), 50 mM NaCl, and 5 mM EDTA), 0.8 ml of 5 M NaCl solution and 8 ml of cold ethanol. The mixture was allowed to stand at -20°C overnight and centrifuged. The precipitate was dissolved in 0.4 ml of TE buffer solution (10 mM Tris-HCl buffer solution (pH 8.0), and 1 mM EDTA) and dialyzed against the same buffer solution to give a crude plasmid.

Bacillus subtilis JB-1-168 (IFO-14144) was transformed with the crude plasmid derived from the chloramphenicol resistant S. epidermidis FS5030 by a protoplast method and the chloramphenicol resistant transformant was isolated (grown in 12.5 μg chloramphenicol/ml). Since the plasmid grew and expressed the gene thereof in the genus Bacillus, it was revealed that the plasmid was usable as a vector for the genus Bacillus. The strain having a chloramphenicol resistanice was isolated among the transformants and named B. subtilis T23.

The B. subtilis T23 was cultured with shaking at 28 °C for 10 hours in L medium. The cells were collected by centrifugation of 500 ml of the obtained culture solution. Thereto were added 60 ml of TES buffer solution containing 25% of sucrose, 12 ml of 0.25 M EDTA (pH 8.0), 16 ml of 5 mg/ml lysozyme solution and 0.8 ml of 5 mg/ml RNase A solution, followed by incubation at 37 °C for 30 min. Then 8 ml of 10% sodium lauryl sulfate was added, followed by further incubation at 37 °C for 15 min. Then 20 ml of 5 M NaCl solution was added and the mixture allowed to stand at 0 °C for 3 hours and then centrifuged. Two volumes of cold ethanol was added to the supernatant. After allowing at -20 °C overnight, the mixture was centrifuged. The precipitate obtained was dissolved in 8.6 ml of TES buffer solution containing 0.4% of Sarkosyl, to which 9 g CsCl and 0.25 ml of 30 mg/ml ethidium bromide solution were added. The resulting mixture was centrifuged at 38,000 rpm at 20 °C for 48 hours by means of a Beckmann ultracentrifuge (rotor 50 Ti). The plasmid band detected by UV irradiation was collected, and mixed with a CsCl-ethidium bromide solution (specific gravity = 1.6), and the mixture centrifuged at 38,000 rpm for 48 hours. The plasmid band was collected, followed by the extraction with n-butanol for removing ethidium bromide and dialysis against the TE buffer solution to give chloramphenicol resistant plasmid pBTM103. The absorbance at 260 nm showed that about 75 μg of the plasmid was obtained.

ii) Construction of cloning vector:

4.5 μg of the pBTM103 was completely digested by restriction enzyme HpaII at 37 °C for 60min. Meanwhile 6.75 μg of pUB110 [J. Scheer - Abramowitz et al., Plasmid, 6, 67 (1981)] was digested by restriction enzyme HpaII at 37 °C for 1 hour. Both reaction mixtures were heat treated at 65 °C for 15 min to stop the reactions. Then precipitation was effected with ethanol. Both of the precipitates were respectively dissolved in 35 ml of water and combined. The mixture was reacted in the presence of 66 nmole of ATP and 10 units of T4 DNA ligase at 11 °C for 31 hours. Ethanol precipitation was then conducted and the precipitate was dissolved in 50 μl of TE buffer solution. 25 μl of the solution was used for transformation of B. subtilis JB-1-168. The strain of B. subtilis T39 having resistance for both kanamycin and chloramphenicol was isolated among the transformants and cultured. 500 ml of the culture solution was treated in the same manner as described in the Reference Example 1-i.

to obtain conjugated plasmid pPBTM119 (209 μg). The molecular weight of the plasmid pBTM119 was about 3.25 M dalton and had a cleavage map as shown in Fig. 1.

Reference Example 2

Construction of Promoter Cloning Vector pBTM126:

A plasmid pBTM126 was constructed by the method of Williams et al. [J. Bacteriol., 146, 1162(1981)] in the following manner. DNA was prepared from *Bacillus pumilus* NCIB 8600 (IFO-12089) provided by The Institute for Fermentation, Osaka, and the DNA (6.5 μg) was cleaved by treatment with 40 units of the restriction enzyme EcoRI at 37 °C for 1 hour, followed by heating at 68 °C for 15 min and the subsequent precipitation with ethanol. Separately, the plasmid pUB110 (20 μg) was cleaved by treatment with 20 units of the restriction enzyme EcoRI at 37 °C for 1 hour, followed by heating at 68 °C for 15 min and precipitation with ethanol. Both the precipitates were respective by dissolved in water and the solutions were combined, 60 nmole of ATP, 10 units of T4 DNA ligase (Takara Shuzo, Japan) and ligase buffer solution were added thereto, and the mixture (100 μl) was maintained at 11 °C for 30 hours. Ethanol precipitation was conducted and the precipitate dissolved in TE buffer solution (50 μl), and 25 μl of the solution used for transformation of *Bacillus subtilis* MI114 [Gene, 24, 255(1983)]. A plasmid was prepared from a chloramphenicol resistant transformant and named pBTM124. Then, the plasmid pBTM124 (2.5 μg) was

cleaved by treatment with 14 units of the restriction enzyme PstI at 37°C for 1 hour, followed by heat treatment at 68 °C for 15 min and ethanol precipitation. The precipitate was dissolved in water, 66 nmol of ATP, 10 units of T4 DNA ligase (Takara Shuzo, Japan) and ligase buffer solution were added, and the resulting mixture (100 µl) was maintained at 11 °C for 24 hours, followed by precipitation with ethanol. The precipitate was dissolved in TE buffer solution and used for transformation of Bacillus subtilis MI114. A plasmid was prepared from one of the resulting kanamycin resistant transformant and named pBTM125. This plasmid was lacking in the chloramphenicol acetyl transferase (CAT) gene promoter region (PstI fragment) of the plasmid pBTM124. Then, the plasmid pBTM125(2.5 µg) was cleaved by treatment with 18 units of the restriction enzyme BamHI and 15 units of the restriction enzyme BglII at 37 °C for 1 hour, followed by heating at 68 °C for 15 min and precipitation with ethanol. The precipitate was dissolved in water and maintained in a reaction medium (100 µl) containing 66 nmole of ATP, 13 units of T4 DNA ligase (Takara Shuzo) and ligase buffer solution at 11 °C for 28 hours, and used for transformation of Bacillus subtilis MI114. A plasmid was prepared from one of the resulting kanamycin resistant transformant and named pBTM126.

Example 1

Cloning of Neutral Protease Gene:

A chromosomal DNA was prepared from B. amyloliquefaciens (IFO-14141) [Institute for Fermentation, Osaka, Research Communication No.11 (1983)] by a per se known method [Methods in Enzymology, 68, 342(1979)]. The chromosomal DNA (8.8 μg) was cleaved by treatment with 36 units of the restriction enzyme BglII at 37°C for 50 min, followed by heating at 65°C for 15min and the subsequent precipitation with ethanol. Separately, the cloning vector pBTM119(2.9 μg) prepared in Reference Example 1 was treated with 30 units of BglII at 37°C for 50 min, followed by adding 0.5 units of alkaline phosphatase, allowing at 65°C for 30 min, and extracting with phenol and with ether and the subsequent precipitation with ethanol. Both the precipitates were respectively dissolved in water and the solutions were combined, to which 66 nmol of ATP, 28 units of T4DNA ligase (Takara Shuzo, Japan) and ligase buffer solution were added, and the mixture (100 μl) was maintained at 11°C for 32 hours. Ethanol precipitation was conducted and the precipitate dissolved in TE buffer solution. Transformation of Bacillus subtilis 1A274 was effected with the use of the resulting solution. A strain forming a halo on the plate of CP agar medium (0.2% yeast extract, 1% pepton, 0.2% NaCl, 1% casein, 1.5% agar, pH7.3) was selected among the chloramphenicol resistant transformants and the plasmid isolated from the strain was named pBTM127. This plasmid had the BglII fragment (3.9 Kb) containing the neutral protease gene of B. amyloliquefaciens (IFO-14141) inserted at the BglII site in the plasmid pBTM119 (see Fig. 2).

The strain of B. subtilis 1A274/pBTM127 carrying the plasmid pBTM127 is deposited with The Institute for Fermentation, Osaka, under IFO-14306.

Example 2

Production of Neutral Protease:

B. amyloliquefaciens (IFO-14141), B. subtilis 1A274 carrying pBTM119 (B. subtilis 1A274/pBTM119] and B. subtilis 1A274 carrying pBTM127 [B. subtilis 1A274/pBTM 127] were each cultivated with shaking in a 200ml Erlenmeyer flask containing 40 ml of CP medium (pH 7.3) including 0.2% yeast extract, 1% peptone, 0.2% NaCl, 1% casein at 28 °C for 24 hours and 48 hours. 5 µg/ml of chloramphenicol was added to the media other than the one for B. amyloliquefaciens. Each culture solution thus obtained was centrifuged and the supernatant was dialyzed against water for a day to obtain an enzyme solution. The protease activity was measured at pH 7 and pH 10 by the method of Miyata et al. [Agric. Biol. Chem., 34, 310(1970)] and the results were as shown in Table 1.

Table 1. Extracellular Protease Activity of strains.

| Strain | Culturing time | | | |
|---|---|---|---|---|
| | 24 hours | | 48 hours | |
| | pH 7 | pH 10 | pH 7 | pH 10 |
| B. amyloliquefaciens (IFO-14141) | 8.9 | 1.7 | 15.1 | 1.7 |
| B. subtilis 1A274/pBTM119 | 0 | 0 | 0 | 0 |
| B. subtilis 1A274/pBTM127 | 136 | 2.6 | 165 | 5.0 |

The unit is unit/ml. supernatant

As shown in Table 1, the ability of B. subtilis 1A274/pPTM127 to produce a neutral protease is more than ten times as much as that of B. amyloliequefaciens (IFO-14141).

Then, a neutral protease was purified to be almost homogeneous from the supernatant of a culture solution obtained from the mass culturing of B. subtilis 1A274/pBTM127 by the fractionation with ammonium sulfate, DEAE cellulose column chromatography and gel filtration with Sephadex G-100.

To the purified sample was added 2% trichloroacetic acid and the mixture was dialyzed against water, followed by lyophilization to obtain powder. The powder obtained was oxidized by performic acid according to the method of Hearth [Methods in Enz., 11. 197 (1967)] and its amino acid sequence at N-terminal was determined by the method of automated Edman degradation [Iwanaga. et al., Eur. J. Biochem. 8, 189 (1969)] using the gas-phase protein sequencer (Applied Bio system Co., USA, model 470A). Phenylthiohydantoin amino acid was determined and analyzed quantitatively by high speed liquid chromatgraphy using a micropack SP-ODS column (Varian, USA). The amino acid sequence at N-terminal (from 1 to 13 residues) was as follows:

1 - 2 - 3 - 4 - 5 - 6 - 7 - 8 - 9 - 10 -11 -12 -13 -
Ala-Ala-Thr-Thr-Gly-Thr-Gly-Thr-Thr-Leu-Lys-Gly-Lys

The above sequence is completely identical with the amino acid sequence of a neutral protease from B. subtilis reported by R.L. Levy et al [Proc. Natl. Acad. Sci., USA, 72, 4341(1975)]

From the above results it is apparent that the gene cloned in Example 1 is of a neutral protease.

Example 3

Subcloning of Neutral Protease Gene:

The plasmid pBTM127 (80 μg) obtained in Example 1 was cleaved with the restriction enzyme BglII (200 units) at 37 °C for 1 hour, followed by 0.7 % agarose gel electrophoresis. The band of a gel showing the smaller molecular weight was recovered. About 10 μg of BglII fragment (3.9 Kb) was obtained by recovering DNA in the said gel by the electrophoresis elution method [Takagi, Manual of Gene Manupilation, Kodansha Scientific, (1982)]. The BglII fragment thus obtained (3 μg) was cleaved with

the restriction enzyme HindIII (6 units) at 37 °C for 1 hour into A-fragment (BglII-HindIII, about 0.8 Kb), B-fragment (Hind III-HindIII, about 1.1 Kb) and C-fragment (HindIII-BglII, about 1.9 Kb), followed by precipitating with ethanol. Separately, the plasmid pBTM126(1 µg) was cleaved by the restriction enzyme HindIII(5 units) at 37 °C for 1 hour, followed by precipitating with ethanol. Both the precipitates were dissolved in water and 100 nmol of ATP, 8.4 units of T4DNA ligase (Takara Shuzo, Japan) and ligase buffer solution were added thereto, and the mixture (100 µl) was maintained at 11 °C, for 24 hours and used for transformation of B. subtilis 1A274. Upon preparation of plasmids from the kanamycin resistant and chloramphenicol sensitive transformants, there were obtained two types of plasmids named, respectively, pBTM508 (6.2 Kb)and pBTM515 (7.7 Kb). The plasmid pBTM515 had the A-fragment and C-fragment inserted at the HindIII site of the plasmid pBTM126 with opposite directions to each other, while the plasmid pBTM508 had the B fragment inserted at the HindIII site of the plasmid pBTM126 (see Fig. 3). The ability of strains carrying these plasmids to form a halo on a CP medium plate, was tested to reveal that the strain carrying pBTM508 had such an ability while the strain carrying pBTM508 did not exhibit such an ability.

Then, the plasmid pBTM127 (1 µg) was cleaved with EcoRI(3 units) and BglII(2 units) and the plasmid pUB110 (1 µg) was cleaved with EcoRI(3 units) and BamHI(5 units) at 37 °C for 1 hour, followed by heating at 65 °C for 10min for the termination of the reaction. Then, ethanol was added to each reaction mixture for causing precipitation. Both the precipitates were dissolved in water and combined, to which were added 100 nmol of ATP, 8.4 units of T4DNA ligase (Takara Shuzo, Japan) and ligase buffer solution. The resultant mixture (100 µl) was maintained at 11 °C for 24 hours and used for transformation of B. subtilis 1A274. The strain having an ability to form halo was selected among the kanamycin resistant transformants and the plasmid from the strain was named pBTM523. The plasmid pBTM523 had the EcoRI-BglII fragment of the plasmid pBTM127 inserted at the EcoRI-BamHI region of the plasmid pUB110 (see Fig. 4). The EcoRI-BglII fragment is composed of a part of the B-fragment and the C-fragment of the BglII fragment of the plasmid pBTM127.

These results revealed that the neutral protease gene exists in the C-fragment (1.9 kb).

The plasmid pBTM515 (100 µg) was cleaved by the restriction enzymes BglII (200 units) and HindIII(200 units) at 37 °C for 1 hour, followed by 1% agarose gel electrophoresis. About 10 µg of the C-fragment was recovered as DNA of 1.9 Kb from the gel by the electrophoresis elution method [vide supra].

The restriction enzyme cleavage map of the C-fragment was as shown in Fig.5.

B. subtilis 1A274 (Bacillus subtilis 1A274/pBTM515) carrying the plasmid pBTM515 has been deposited with IFO under IFO-14377 and also with Fermentation Research Institute, Agency of Industrial Science and Technology, Ministry of International Trade and Industry (FRI) under FERM BP-622 according to the Budapest Treaty.

Example 4

The base sequence of a part of the C-fragment obtained in Example 3 was determined by the dideoxynucleotide synthetic chain termination method and the method of Maxam-Gilbert. The base sequence from the BglII site in the C-fragment and the amino acid sequence translated therefrom are shown in Fig. 6. In the base sequence, the -35 region (TTGCAG) and -10 region (TATTAT) recognized by $E\sigma^{55}$ as a promoter, the -35 region (AGTTTT) and -10 region (GAGATTGCT) recognized by $E\sigma^{37}$ and the -35 region (AATTC) and -10 region (TAGTTTTATA) which presumably recognized by $E\sigma^{32}$ can be seen. Since the fact that the BglII-Sau3A1 fragment (530 bp) (see Fig. 5) has a promoter activity has been confirmed by using the promoter cloning vector pFTB 281 [Yoshimura et al, Abstracts of Papers, Annual Meeting of the Agricultural Chemical Society of Japan, 1983, pp28 : Yoshimura et al, J. Bacteriol. 159, 905 (1984)], the above-mentioned promoter is considered to function. The SD region and an open frame exist downstream from the promoter. The amino acid sequence from the 1st Met to 27th or 28th Ala appears to be a signal peptide of the neutral protease. While the amino acid sequence after 222th Ala slightly differs from that of the neutral protease reported in the article [P. L. Levy et al, Proc, Natl. Acad. Sci., 72, 4341 (1975)], it is completely identical with the amino acid sequence at N-terminal of the neutral protease shown in Example 3. These results suggest the presence of a peptide having about 200 amino acids and an unknown function between the sequence expected to be a signal peptide and the mature protein.

The base sequence from the HindIII site of the C-fragment and the amino acid sequence translated therefrom are shown in Fig. 7. This amino acid sequence is completely identical with the amino acid sequence from the C-terminal of a neutral protease reported in the above article.

Example 5

10 μg of BglII-HindIII fragment (1.9 Kb) obtained in Example 3 were cleaved with the restriction enzyme AccI(50 units) at 37 °C for 1 hour, followed by 1.2% agarose gel electrophoresis. The band of the gel containing the BglII-AccI fragment (1.7 Kb) was cut out and subjectid to a electroelution, extraction with phenol and ether and precipitation with ethanol, thereby to obtain 3 μg of the BglII-AccI fragment. Upon Determining the base sequence of the fragment by the method of Maxam-Gilbert [Proc. Natl. Acad. Sci., 74, 560 (1977)], the region of the structural gene of a neutral protease, encoding for 30 amino acids located from the C-terminal, was found to be missing.

Example 6

There is a cleavage site, GCATGC, for the restriction enzyme SphI near the DNA coding for the N-terminal of the mature protein of a neutral protease (see Fig. 6). A plasmid pTEX101, namely the plasmid pUB110 inserted with the neutral protease gene being deficient in the region coding for the mature protein, was constructed by the following method.

The BglII-SphI fragment (0.9 Kb) isolated from the plasmid pBTM515 and the BamHI-SphI fragment (4.3 Kb) isolated from the plasmid pUB110 were ligated with T4 DNA ligase, and the product obtained was used for the transformation of B. subtilis 1A274. A plasmid was prepared from the kanamycin resistant transforment and named pTEX101 (see Fig. 8).

Example 7

Construction of Human Immune Interferon Secretion Plasmid pBTM150:

The ClaI-PstI fragment (1.03 Kb) containing the human immune interferon gene isolated from the plasmid pHITtrp2101 (see EPO Laid Open Publication No. 0103898) was cleaved with the restriction enzyme BstNI (New England BioLabs), followed by agarose gel electrophoresis. From the gel was isolated 900bp of BstNI fragment by the electroelution method. The BstNI fragment (2 μg) was

added with oligonucleotides CCTGTTACTGCC (266 ng) and TGGCAGTAACAGGCATG (377 ng) which were chemically synthesized by the tri-ester method (vide supra) and thereafter phosphorylated, to which were further added 100 nmol of ATP, a ligasion buffer solution and 2,000 units of T4 DNA ligase (New England BioLabs). The resulting mixture (50 µl) was then maintained at 11 °C for 24 hours. Ethanol precipitation was conducted and the precipitate was dissolved in water, followed by a treatment with 50 units of the restriction enzyme SphI (New England BioLabs) at 37 °C for 16 hours. After removing excess oligonucleotides by gel filtration using Sephadex 4B (1.5 ml), ethanol was added to effect precipitation.

Separately 0.6 µg of pTEX101 obtained in Example 6 was cleaved by 1 unit of SphI at 37 °C for 1 hour. The resulting mixture, after being added with 0.06 units of E. coli alkaline phosphatase, was maintained at 37 °C for 30 min, followed by precipitation with ethanol.

Both of the thus obtained precipitates were dissolved in water and mixed with each other, to which were added 100 nmol of ATP, a ligase buffer solution and 400 units of T4 DNA ligase (New England BioLabs). The resultant mixture (50 µl) was maintained at 11 °C for 24 hours and the product was used for transformation of B. subtilis MH401 ($met^-$, $his^-$, $npr^-$) derived from B. subtilis 1A274. Plasmids were prepared from the kanamycin resistant transformants and the one with the human immune interferon gene being inserted in the same direction as that of the neutral protease promoter was named pBTM 150 and the other with the human immune interferon gene being inserted in the reverse direction was named pBTM151 (see Fig. 9).

B. subtilis MH401 is deposited with The Institute For Fermentation, Osaka under IFO-14374, and according to the Budapest Treaty, it is also deposited with Fermentation Research Institute, Agency of Industrial Science and Technology, Ministry of International Trade and Industry (FRI) under FERM BP-621.

Example 8

Secreting Expression of Human Immune Interferon Gene

B. subtilis MH401 carrying the plasmids pTEX101, pBTM150 and pBTM151 were each inoculated into a test tube containing 5 ml of L medium composed of 1% pepton (Difco), 0.5% yeast extract, and 0.5% NaCl and cultured at 28 C for 16 hours with shaking. 0.5 ml of each resultant culture solution was transferred to a 200 ml Erlenmeyer flask containing 40 ml of a brain heart infusion medium (Difco) including 1% casein and 5 μg/ml kanamycin for further cultivation at 37°C for 8 hours with shaking. The resulting solution was centrifuged to obtain a supernatant. The cells were washed three times with 1 M KCl and frozen by dried ice-ethanol. The frozen cells were suspended in 0.9 ml of 30 mM Tris-HCl (pH 8.0) - 50

mM NaCl- 5 mM EDTA -0.1 mM phenyl methylsulfonyl fluoride and 0.1 ml of 10 mg/ml lysozyme solution. The mixture was maintained at 37°C for 20 min and then treated by a sonicator (Kaijyo Denki, Japan) at 19.5 KHZ for 10 seconds. The sonicated product was centrifuged at 9,000 rpm for 20 min to obtain as a supernatant an extract of the cells.

The activity of the human immune interferon in the supernatant of the culture solution and the extract of the cells thus obtained was measured by FL sindbis system (an antiviral activity against the virus sindbis inoculated in FL cells) [Kohase et al., Protein, Nucleic Acids And Enzyme, 25, 355(1981)]. At the assay, the international standard of human immure interferon (Gg 23-901-503:4000 IU/ml) was used as a control.

With a strain carrying the plasmid pBTM150, $10^5$ IU/l of human immune interferon was found to be produced in the culture medium, but no human immune interferon activity was detected in the extract of the cells. The human immune interferon activity was not able to be detected in the culture solution or in the extract of the cells, with both of the strain carrying the plasmid pTEX101 having inserted therein no human immune interferon gene and the strain carrying the plasmid pBTM151 having inserted therein the human immune interferon gene in the reverse direction of the neutral protease promoter. It becomes apparent from the above results that the human immune interferon gene is expressed for secretion by pBTM150.

Example 9

Construction of Penicillinase Secretion Plasmid

A plasmid pTR10, which was constructed by inserting a BglII-Sau3AI fragment (about 530 bp) containing the neutral protease promoter, the region coding for a signal peptide and the region coding for upstream side of a propeptide the C-fragment of a plasmid pBTM515 into the BamHI site of a pFTB281, was cleaved with EcoRI to obtain an EcoRI fragment (about 530 bp). By cleaving the fragment with the restriction enzyme HaeIII, an EcoRI-HaeIII fragment (about 340 bp) including the promoter and a region coding for the signal peptide up to near its C-terminal was obtained. A XhoI linker (CCTCGAGG) was ligated with the fragment by T4 DNA ligase.

Separately, a SacI-PstI fragment (8.5 Kb) was isolated from the plasmid pEN 1 (obtained from Prof. Oshima, Osaka Univ. Japan) (see Fig. 10) which was constructed by inserting the penicillinase gene of <u>Bacillus licheniformis</u> into the plasmid pMB9 [F. Bolivar et al., Gene <u>2</u>, 75(1977)]. This fragment has a region coding for the latter half of the signal peptide of the penicillinase and for the mature protein and the PstI site is located in the middle of the region coding for the signal peptide. Then, the SacI-PstI fragment was treated with 5 units of nuclease BAL31 (Bethesda Research Laboratories) at room temperature for 15 seconds followed by the ligation of an XhoI linker (CCTCGAGG, New England BioLabs) using T4 DNA ligase. The product was used for transformation of E.coli JA221 (obtained from Prof. Oshima, Osaka Univ.). The size of a BglII-XhoI fragment of the plasmid of the tetracycline resistant transformant was measured by polyacrylamide gel electrophoresis and several clones in which a greater part of the region coding for the signal peptide was thought to be missing were obtained. The base sequence of each of these clones was determined by the dideoxynucleotide synthetic chain termination method. One of such clones, the plasmid pENX374 (8.3 Kb) was found to contain a nucleotide coding for Ser-Arg-Ala and linked at the 5' end of the DNA coding for a mature protein of penicillinase (see Figs. 10, 11 and 12).

In the meantime, pUB110 was cleaved by the restruction enzymes SphI and EcoRI and a large SphI-EcoRI fragment (3.5 Kb) was isolated by agarose gel electrophoresis and electroelution (see Fig. 14).

The thus obtained three fragments, namely the EcoRI-XhoI fragment (340 bp, 0.5 μg) containing the neutral protease promoter and its signal peptide encoding region, the XhoI-SphI fragment (1.9 Kb, 0.5 μg) derived from pENX374 and the SphI-EcoRI fragment (0.5 μg) derived from pUB110 were ligated by T4 DNA ligase. Using the ligated product transformation of B.subtilis 1A274 was conducted and a plasmid having all of these fragments was selected among the kanamycin resistant transformants and was named pTEX201(5.7 Kb) (see Fig. 14).

Example 10

Secreting Expression of a Penicillinase Gene

*B. subtilis* 1A274 carrying the plasmids pUB110 and pTEX201 were each cultured with shaking in L medium including 1% casein at 37°C for 24 hours and the penicillinase activity of the supernatant of each culture solution was measured by the micro-iodine method [Okonogi et al, Chemotherapy, 25, 94(1977)]. With the plasmid pTEX201, 2.1 x $10^4$ units/l culture solution of penicillinase were produced in the supernatant, while with the plasmid pUB110 free of the penicillinase gene, almost no penicillinase was found to be produced. These results showed that a strain carrying the plasmid pTEX201 was able to produce penicillinase extracellularly.

*B. subtilis* 1A274 carrying the plasmid pTEX201 (*Bacillus subtilis* 1A274/pTEX201) is deposited with The Institute For Fermentation, Osaka under IFO-14375 and, according to the Budapest Treaty, it is also deposited with Fermentation Research Institute, Agency of Industrial Science and Technology, Ministry of International Trade and Industry (FRI) under FERM BP-619.

Example 11

Construction of a Penicillinase Secretion Plasmid

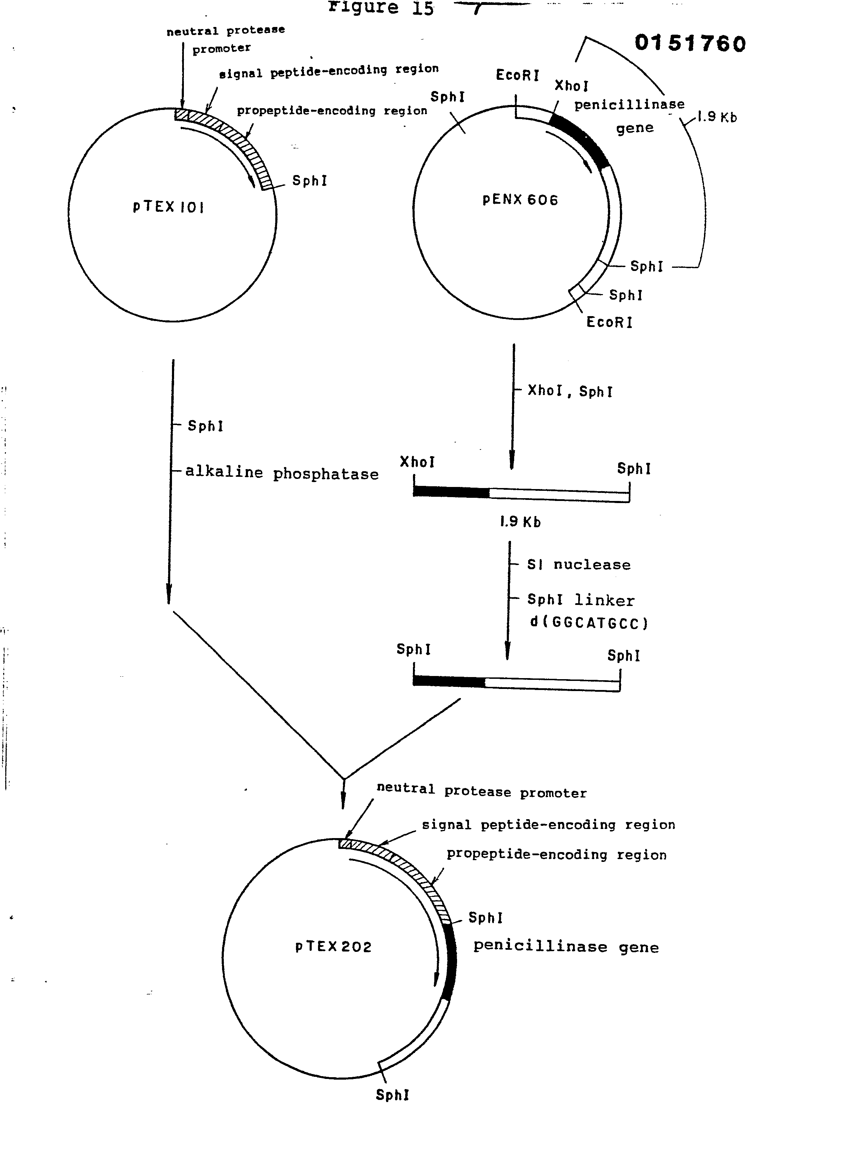

In the same manner as described in Example 9, the SacI-PstI fragment of pEN1 was treated with BAL31, followed by ligating of the XhoI linker to obtain the plasmid pENX606. In the plasmid pENX606, 40 nucleotides from 5' end were missing in the region coding for the mature protein of a penicillinase in comparison with the plasmid pENX374 (see Fig. 11 and 13). The XhoI-SphI fragment (1.9 Kb) including the region encoding for the mature protein of a penicillinase was isolated from the plasmid pENX606 and 2 μg of the fragment was treated with 100 units of S1 nuclease (Bethesda Research Laboratories) at 15 °C for 1 hour followed by ligation of the SphI linker (GGCATGCC) using T4 DNA ligase. The SphI fragment (1.9 Kb) thus obtained was inserted into the SphI site of the pTEX101 to obtain the penicillinase secretion vector pTEX202 (see Fig. 15).

Example 12

Secreting Expression of Penicillinase Gene

*B. subtilis* 1A274 carrying the plasmid pTEX202 (*Bacillus subtilis* 1A274/pTEX202) was cultured with shaking in L medium including 1% casein at 28 °C for 6 hours. The penicillinase activity of the supernatant obtained by centrifugation was measured by the micro-iodine method (vide supra) to show a penicillinase activity of 5.0 x $10^3$ units/l culture solution.

*Bacillus subtilis* 1A274/pTEX202 is deposited with Institute For Fermentation, Osaka under IFO-14378 and, according to the Budapest Treaty, it is also deposited with Fermentation Research Institute, Agency of Industrial Science and Technology, Ministry of International Trade and Industry (FRI) under FERM BP-620.

## CLAIMS

1. A DNA comprising a promoter, and the neutral protease gene containing a signal peptide-encoding region and having at least a portion of its neutral protease-encoding region cut away, said cut away portion being the 3' end nucleotide, or the 3' end nucleotide and one or more contiguous necleotides linked thereto.

2. A DNA as claimed in claim 1, wherein said signal peptide contains a peptide having the following amino acid sequence:
Met-Gly-Leu-Gly-Lys-Lys-Leu-Ser-Val-Ala-Val-Ala-Ala-Ser-Phe
-Met-Ser-Leu-Thr-Ile-Ser-Leu-Pro-Gly-Val-Gln-Ala

3. A DNA as claimed in claim 1, wherein said promoter is a neutral protease promoter.

4. A DNA as claimed in claim 1, wherein the -35 and -10 regions of said promoter include the nucleotides represented by TTGCAG and TATTAT, respectively.

5. A DNA as claimed in claim 1, wherein the -35 and -10 regions of said promoter include the nucleotides represented by AGTTTT and GAGATTGCT, respectively.

6. A DNA as claimed in claim 1, wherein the -35 and -10 regions of said promoter include the nucleotides represented by AATTC and TAGTTTTATA, respectively.

7. A DNA as claimed in claim 1, wherein said promoter has a polynucleotide chain having no more than 100 bases and regions with base sequences represented by TTGCAG, TATTAT, AGTTTT, GAGATTGCT, AATTC and TAGTTTTATA.

8. A DNA as claimed in claim 7, wherein said polynucleotide chain has the nucleotides AATTC and TATTAT at its 5' and 3' ends, respectively.

9. A DNA as claimed in claim 8, wherein said polynucleotide chain contains the polynucleotide represented by
AATTCAGCCGCGGAGTCTACTTTTATATTGCAGAATGCGAGATTGCTGGTTTATTAT.

10. A DNA as claimed in claim 1, which is pTEX101.

11. A DNA as claimed in claim 1, which further contains a structural gene coding for a protein other than a neutral protease.

12. A DNA as claimed in claim 11, wherein said neutral protease gene is located downstream from said promoter with said structural gene being located downstream from said neutral protease gene.

13. A DNA as claimed in claim 12, which is pBTM150.

14. A DNA as claimed in claim 12, which is pTEX201.

15. A DNA as claimed in claim 12, which is pTEX202.

16. A method of producing a DNA, comprising (a) digesting a first DNA fragment containing the neutral protease gene having a promoter, a signal peptide-encoding region and a neutral protease-encoding region with a restriction enzyme to cut away a portion of the neutral protease-encoding region, said cut away portion being the 3' end nucleotide, or the 3' end nucleotide and one or more contiguous necleotides linked thereto; or (b) joining a second DNA fragment having promoter activity to a third DNA fragment containing a signal peptide-encoding region of the neutral protease gene and a neutral protease-encoding region, at least a portion of said neutral protease-encoding region being missing, said missing portion being the 3' end nucleotide, or the 3' end nucleotide and one or more contiguous nucleotides linked thereto; and, if necessary, (c) joining a desired protein-encoding gene at a position downstream from said signal peptide-encoding region, whereby obtaining a DNA comprising a promoter, and the neutral protease gene containing a signal peptide-encoding region and having at least a portion of its neutral protease-encoding region cut away, said cut away portion being the 3' end nucleotide, or the 3' end nucleotide and one or more contiguous necleotides linked thereto, optionally having the desired protein-encoding gene at a position downstream from said signal peptide-encoding region.

17. A method as claimed in claim 16, wherein said signal peptide contains a peptide having the following amino acid sequence:

Met-Gly-Leu-Gly-Lys-Lys-Leu-Ser-Val-Ala-Val-Ala-Ala-Ser-Phe-Met-Ser-Leu-Thr-Ile-Ser-Leu-Pro-Gly-Val-Gln-Ala

18. A method as claimed in claim 16, wherein said first DNA fragment is derived from a chromosome of a microorganism capable of producing a neutral protease.

19. A method as claimed in claim 16, wherein said first DNA fragment is derived from a chromosome of a strain of microorganism belonging to the genus Bacillus

20. A method as claimed in claim 16, wherein said first DNA fragment has the restriction enzyme cleavage map illustrated in Fig. 5 of the accompanying drawings.

21. A method as claimed in claim 16, wherein said second DNA fragment includes a neutral protease promoter.

22. A method as claimed in claim 16, wherein said desired protein is human interferon or penicillinase.

23. A method as claimed in claim 16, wherein the DNA product is pTEX101.

24. A method as claimed in claim 16, wherein the DNA product is pBTM150, pTEX201 or pTEX202.

25. A transformant of a strain of microorganism belonging to the genus Bacillus, which contains a DNA according to claim 1.

26. A transformant as claimed in claim 25, wherein said DNA further contains a gene coding for a protein other than a neutral protease.

27. A transformant as claimed in claim 26, which is Bacillus subtilis MH401/pBTM150.

28. A transformant as claimed in claim 26, which is Bacillus subtilis 1A274/pTEX201.

29. A transformant as claimed in claim 26, which

is Bacillus subtilis 1A274/pTEX202.

30. A process for the production of a protein, which comprises cultivating in a medium a transformant of a strain of microorganism belonging to the genus Bacillus, which contains a DNA including a promoter, the neutral protease gene containing a signal peptide-encoding region, and a gene coding for a protein other than the neutral protease, said neutral protease gene having at least a portion of its neutral protease-encoding region cut away, said cut away portion being the 3' end nucleotide, or the 3' end nucleotide and one or more contiguous nucleotides linked thereto, whereby said peptide is secreted into the medium, and recovering said protein from the medium.

31. A process as claimed in claim 30, wherein said neutral protease gene is located downstream from said promoter with said structural gene being located downstream from said neutral protease gene.

32. A process as claimed in claim 30, wherein said transformant is Bacillus subtilis MH401/pBTM150, Bacillus subtilis 1A274/pTEX201 or Bacillus subtilis 1A274/pTEX202.

Figure 1

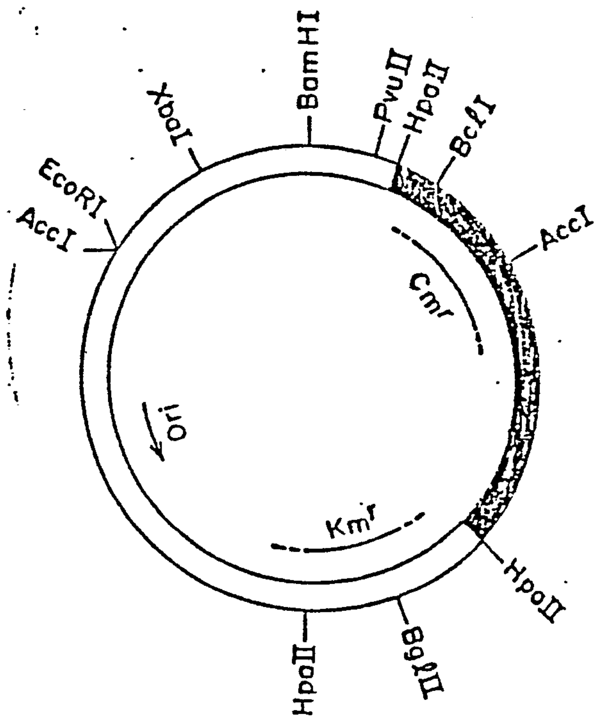
BamHI
PvuII
HpaII
BclI
AccI
XbaI
EcoRI
AccI
Cmr
Ori
Kmr
HpaII
BglII
HpaII

Figure 2

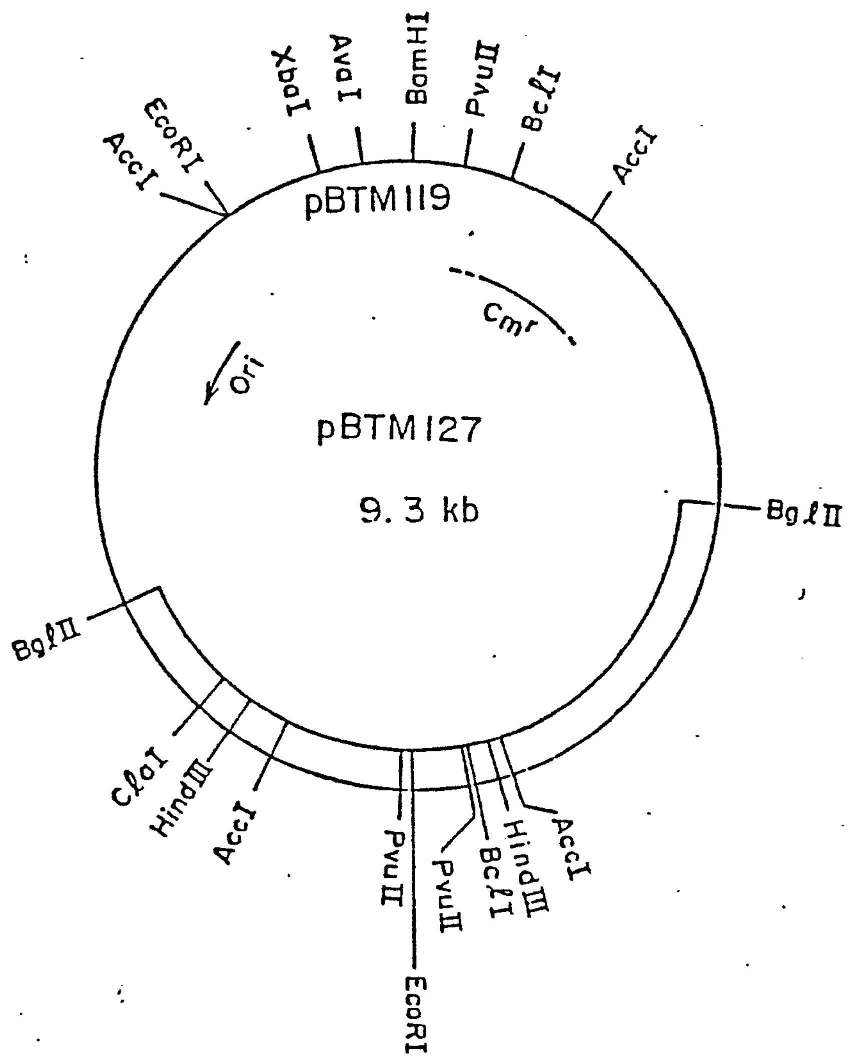
XbaI
AvaI
BamHI
PvuII
BclI
EcoRI
AccI
AccI
pBTM119
Cmr
ori
pBTM127
9.3 kb
BglII
BglII
ClaI
HindIII
AccI
PvuII
PvuII
BclI
HindIII
AccI
EcoRI

Figure 3

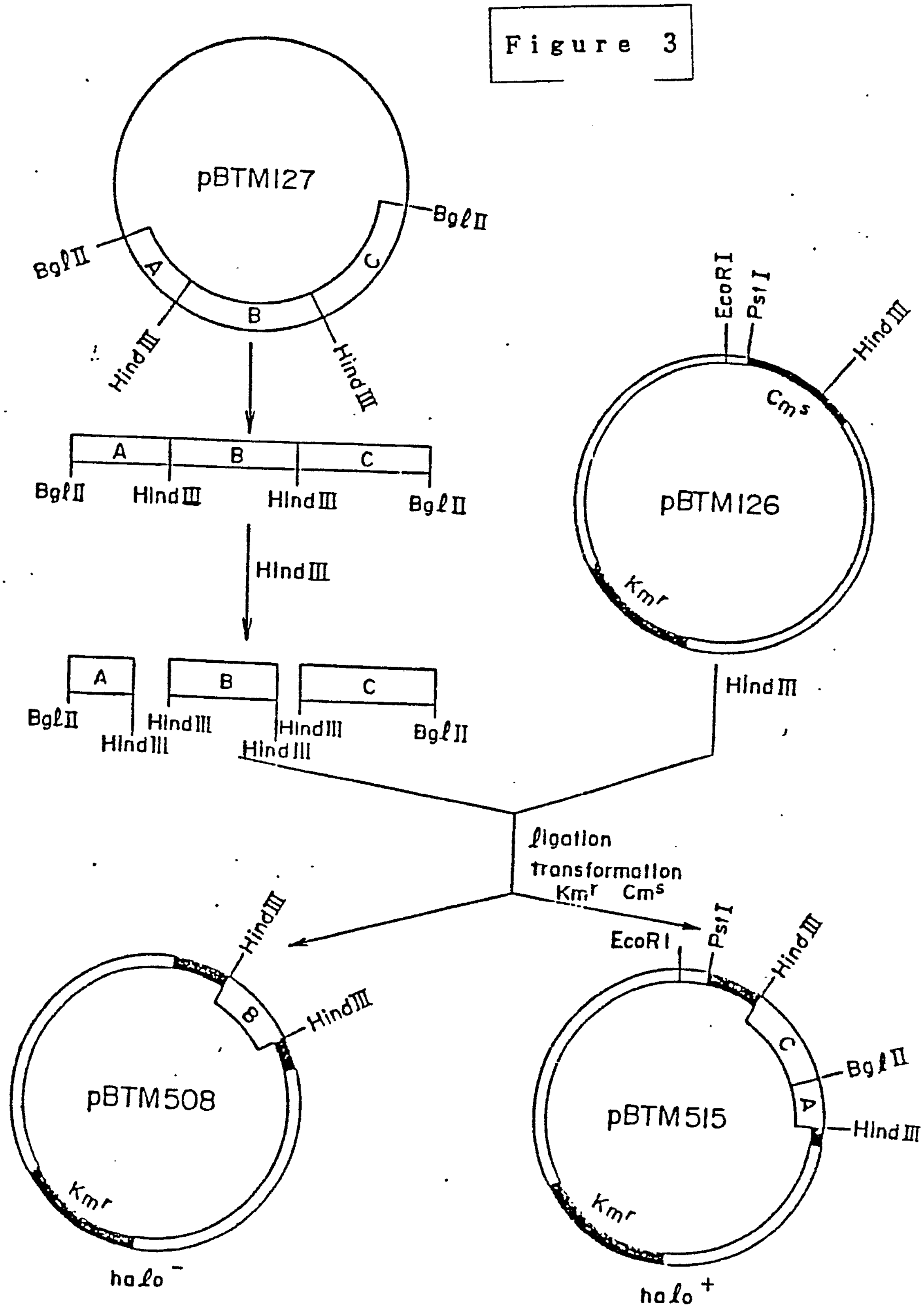
pBTM127
BglII
A
B
C
HindIII
HindIII
BglII
A
B
C
BglII
HindIII
HindIII
BglII
HindIII
A
B
C
BglII
HindIII
HindIII
HindIII
HindIII
BglII
EcoRI
PstI
HindIII
Cms
pBTM126
Kmr
HindIII
ligation
transformation
Kmr Cms
HindIII
B
HindIII
pBTM508
Kmr
halo −
EcoRI
PstI
HindIII
C
BglII
A
HindIII
pBTM515
Kmr
halo +

Figure 4

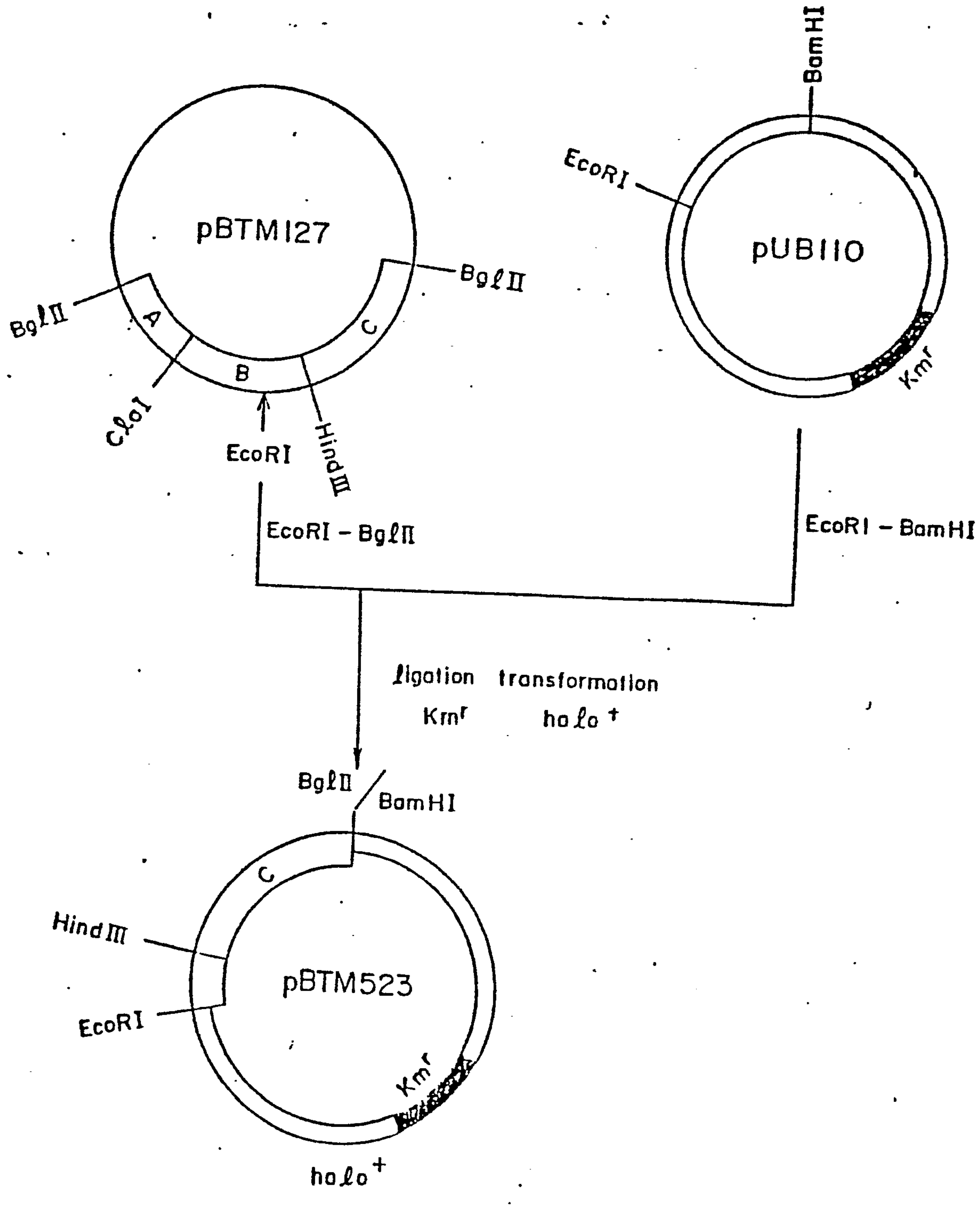
pBTM127
BglII
A
B
C
BglII
ClaI
EcoRI
HindIII
BamHI
EcoRI
pUB110
Kmr
EcoRI - BglII
EcoRI - BamHI
ligation transformation
Kmr halo +
BglII
BamHI
C
HindIII
pBTM523
EcoRI
Kmr
halo +

Figure 5

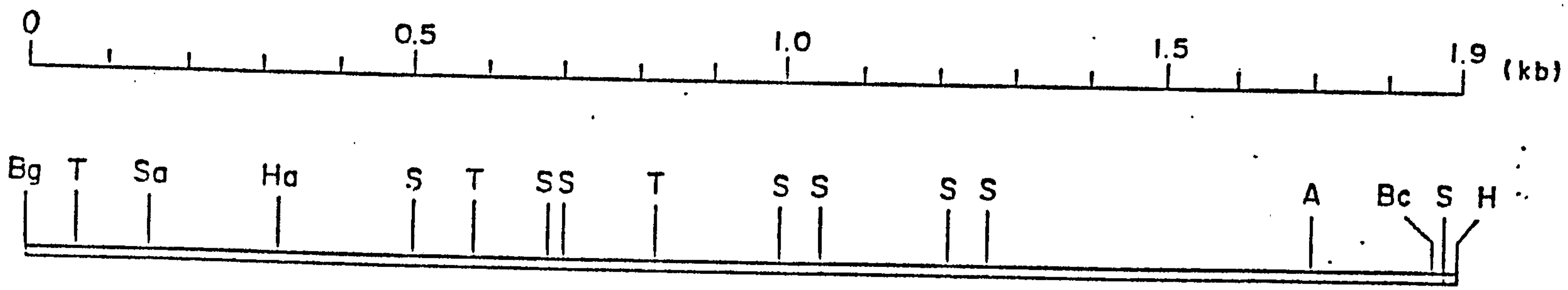

0151760

# Figure 6(a)

```
1     GATCTTAACATTTTTCCCCTATCATTTTTCCCGTCTTCATTTGTCATTTTTTCCAGAAAAAATCGCGTCA

71    TTCGACTCATGTCTAATCCAACACGTGTCTCTCGGCTTATCCCCTGACACCGCCCGCCGACAGCCCGCAT

141   GGGACGATTCTATCAATTCAGCCGCGGAGTCTAGTTTTATATTGCAGAATGCGAGATTGCTGGTTTATTA

211   TAACAATATAAGTTTTCATTATTTTCAAAAAGGGGGATTTATT GTG GGT TTA GGT AAG AAA TTG
1                                                 Met Gly Leu Gly Lys Lys Leu

275   TCT GTT GCT GTC GCC GCT TCC TTT ATG AGT TTA ACC ATC AGT CTG CCG GGT GTT
8     Ser Val Ala Val Ala Ala Ser Phe Met Ser Leu Thr Ile Ser Leu Pro Gly Val

329   CAG GCC GCT GAG AAT CCT CAG CTT AAA GAA AAC CTG ACG AAT TTT GTA CCG AAG
26    Gln Ala Ala Glu Asn Pro Gln Leu Lys Glu Asn Leu Thr Asn Phe Val Pro Lys

383   CAT TCT TTG GTG CAA TCA GAA TTG CCT TCT GTC AGT GAC AAA GCT ATC AAG CAA
44    His Ser Leu Val Gln Ser Glu Leu Pro Ser Val Ser Asp Lys Ala Ile Lys Gln

437   TAC TTG AAA CAA AAC GGC AAA GTC TTT AAA GGC AAT CCT TCT GAA AGA TTG AAG
62    Tyr Leu Lys Gln Asn Gly Lys Val Phe Lys Gly Asn Pro Ser Glu Arg Leu Lys

491   CTG ATT GAC CAA ACG ACC GAT GAT CTC GGC TAC AAG CAC TTC CGT TAT GTG CCT
80    Leu Ile Asp Gln Thr Thr Asp Asp Leu Gly Tyr Lys His Phe Arg Tyr Val Pro

545   GTC GTA AAC GGT GTG CCT GTG AAA GAC TCT CAA GTC ATT ATT CAC GTC GAT AAA
98    Val Val Asn Gly Val Pro Val Lys Asp Ser Gln Val Ile Ile His Val Asp Lys

599   TCC AAC AAC GTC TAT GCG ATT AAC GGT GAA TTA AAC AAC GAT GTT TCC GCC AAA
116   Ser Asn Asn Val Tyr Ala Ile Asn Gly Glu Leu Asn Asn Asp Val Ser Ala Lys

653   ACG GCA AAC AGC AAA AAA TTA TCT GCA AAT CAG GCG CTG GAT CAT GCT TAT AAA
134   Thr Ala Asn Ser Lys Lys Leu Ser Ala Asn Gln Ala Leu Asp His Ala Tyr Lys

707   GCG ATC GGC AAA TCA CCT GAA GCC GTT TCT AAC GGA ACC GTT GCA AAC AAA AAC
152   Ala Ile Gly Lys Ser Pro Glu Ala Val Ser Asn Gly Thr Val Ala Asn Lys Asn
```

<to be continued>

Figure 6(b)

```
761  AAA GCC GAG CTG AAA GCA GCA GCC ACA AAA GAC GGC AAA TAC CGC CTC GCC TAT
170  Lys Ala Glu Leu Lys Ala Ala Ala Thr Lys Asp Gly Lys Tyr Arg Leu Ala Tyr

815  GAT GTA ACC ATC CGC TAC ATC GAA CCG GAA CCT GCA AAC TGG GAA GTA ACC GTT
188  Asp Val Thr Ile Arg Tyr Ile Glu Pro Glu Pro Ala Asn Trp Glu Val Thr Val

869  GAT GCG GAA ACA GGA AAA ATC CTG AAA AAG CAA AAC AAA GTG GAG CAT GCC GCC
206  Asp Ala Glu Thr Gly Lys Ile Leu Lys Lys Gln Asn Lys Val Glu His Ala Ala

923  ACA ACC GGA ACA GGT ACG ACT CTT AAA GGA AAA ACG GTC TCA TTA AAT ATT TCT
224  Thr Thr Gly Thr Gly Thr Thr Leu Lys Gly Lys Thr Val Ser Leu Asn Ile Ser

977  TCT GAA AGC GGC AAA TAT GTG CTG CGC GAT C
242  Ser Glu Ser Gly Lys Tyr Val Leu Arg Asp
```

0151760

Figure 7

```
  1   GAC CTT TAC GGC TCT CAA GAT GCT GCA AGC GTA GAA GCT GCC TGG AAT GCA GTC
  1   Asp Leu Tyr Gly Ser Gln Asp Ala Ala Ser Val Glu Ala Ala Trp Asn Ala Val

 55   GGA TTG TAA ACAAGAAAAGAGACCGGAAATCCGGTCTCTTTTTTATATCTAAAAACATTTCACAGTGG
 19   Gly Leu ***

123   CTTCACCATGATCATATATGTCTTTTCCCGATCGTCTTTTTCAAGCTT
```

Figure 8

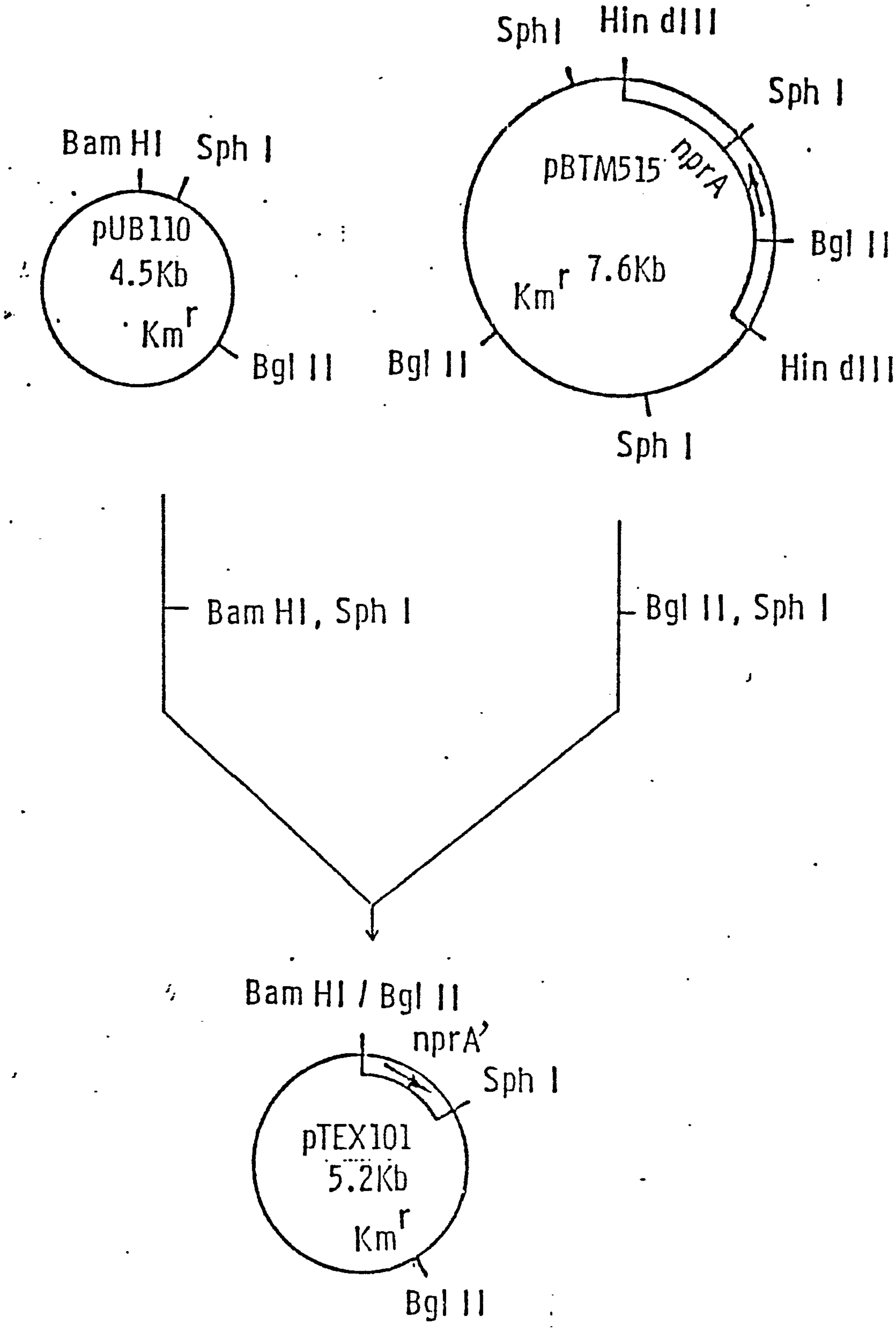
Bam HI
Sph I
pUB110
4.5Kb
Km^r
Bgl II
Sph I
Hin dIII
Sph I
pBTM515
nprA
Bgl II
Km^r 7.6Kb
Bgl II
Hin dIII
Sph I
Bam HI, Sph I
Bgl II, Sph I
Bam HI / Bgl II
nprA'
Sph I
pTEX101
5.2Kb
Km^r
Bgl II

Figure 9

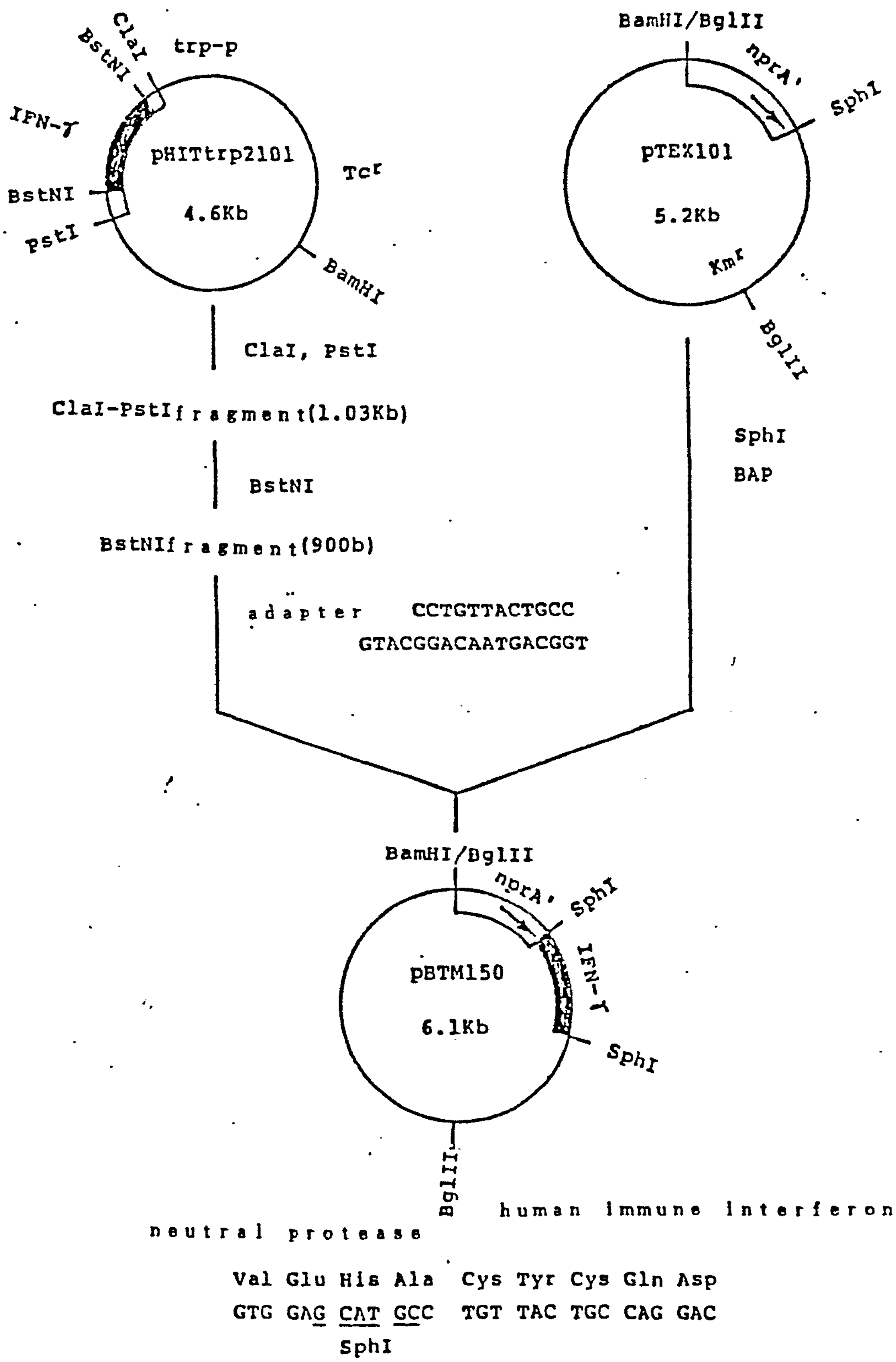
ClaI
BstNI
trp-p
IFN-γ
pHITtrp2101
4.6Kb
Tcr
BstNI
PstI
BamHI
BamHI/BglII
nprA'
SphI
pTEX101
5.2Kb
Kmr
BglII
ClaI, PstI
ClaI-PstI fragment(1.03Kb)
BstNI
BstNI fragment(900b)
SphI
BAP
adapter CCTGTTACTGCC
GTACGGACAATGACGGT
BamHI/BglII
nprA'
SphI
IFN-γ
pBTM150
6.1Kb
SphI
BglII
neutral protease
human immune interferon
Val Glu His Ala Cys Tyr Cys Gln Asp
GTG GAG CAT GCC TGT TAC TGC CAG GAC
SphI

0151760

Figure 10

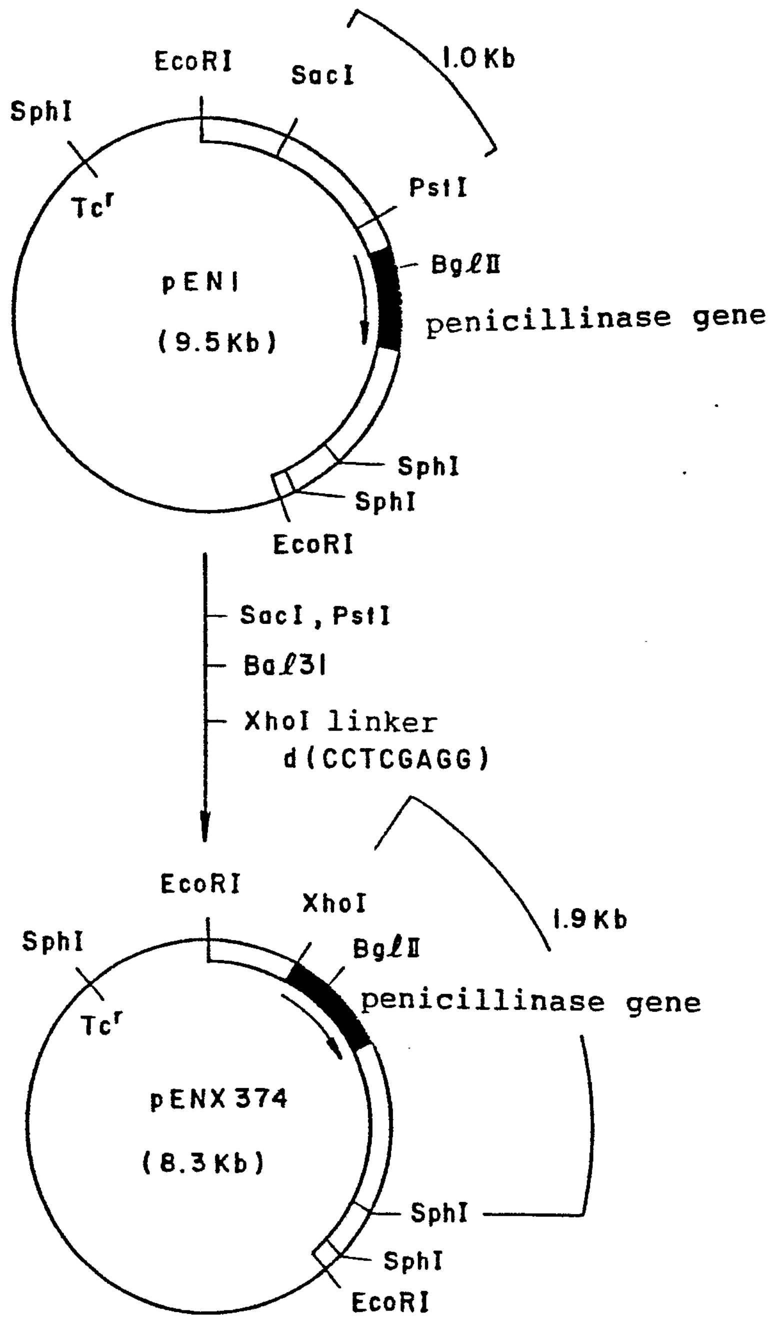

## Figure 11

```
ATATTCAAACGGAGGGAGACGATTTTG ATG AAA TTA TGG TTC AGT ACT TTA AAA CTG AAA
                            Met Lys Leu Trp Phe Ser Thr Leu Lys Leu Lys

        PstI
AAG GCT GCA GCA GTG TTG CTT TTC TCT TGC GTC GCG CTT GCA GGA TGC GCT AAC
Lys Ala Ala Ala Val Leu Leu Phe Ser Cys Val Ala Leu Ala Gly Cys Ala Asn
                                                            Membrane

AAT CAA ACG AAT GCC TCG CAA CCT GCC GAG AAG AAT GAA AAG ACG GAG ATG AAA
Asn Gln Thr Asn Ala Ser Gln Pro Ala Glu Lys Asn Glu Lys Thr Glu Met Lys
                    exo-L                           exo-S
```

## Figure 12

```
TCG AGG GCC TCG CAA CCT GCC GAG AAG AAT GAA AAG ACG GAG ATG AAA
Ser Arg Ala Ser Gln Pro Ala Glu Lys Asn Glu Lys Thr Glu Met Lys
```

## Figure 13

```
T CGA GGG ATG AAA
  Arg Gly Met Lys
```

Figure 14

0151760

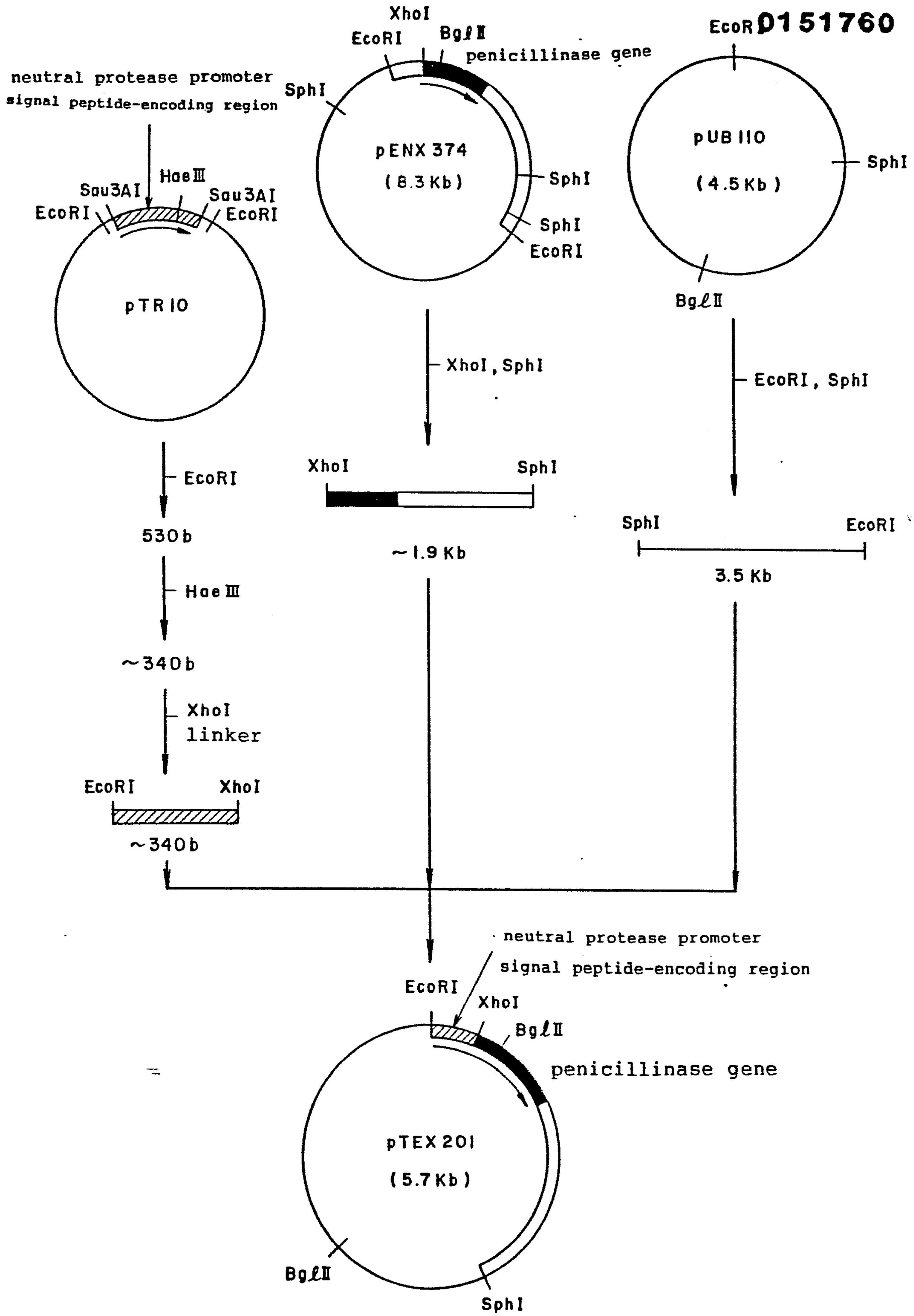

Figure 15

0151760